(19) Europäisches Patentamt
European Patent Office
Office européen des brevets

(11) EP 1 621 185 A1

(12) DEMANDE DE BREVET EUROPEEN

(43) Date de publication:
**01.02.2006 Bulletin 2006/05**

(51) Int Cl.:
*A61K 8/87* *(2006.01)* *A61Q 5/10* *(2006.01)*
*A61K 8/31* *(2006.01)* *A61K 8/34* *(2006.01)*

(21) Numéro de dépôt: **05291420.7**

(22) Date de dépôt: **30.06.2005**

(84) Etats contractants désignés:
**AT BE BG CH CY CZ DE DK EE ES FI FR GB GR HU IE IS IT LI LT LU MC NL PL PT RO SE SI SK TR**
Etats d'extension désignés:
**AL BA HR LV MK YU**

(30) Priorité: **01.07.2004 FR 0451389**

(71) Demandeur: **L'OREAL**
**75008 Paris (FR)**

(72) Inventeurs:
- **Rollat-Corvol, Isabelle**
  **75017 Paris (FR)**
- **Gawtrey, Jonathan**
  **92100 Boulogne (FR)**

(74) Mandataire: **Fevrier, Murielle Françoise E.**
**L'OREAL - D.I.P.I.**
**25-29 Quai Aulagnier**
**92600 Asnières (FR)**

# (54) Composition de coloration comprenant un polymère filmogène élastomère et un précurseur de colorant d'oxydation

(57) L'invention a pour objet une composition de coloration comprenant, dans un milieu approprié à la coloration, au moins un précurseur de colorant et au moins un polymère filmogène élastomère choisi de telle sorte que le film obtenu par séchage de ce polymère, à température ambiante et à un taux d'humidité relative de 55 %, présente un profil mécanique défini par au moins :

(a) un taux d'allongement à la rupture ($\varepsilon_r$) supérieur ou égal à 800 %,

(b) une recouvrance instantanée ($R_i$) au moins égale à 75 %, après un allongement de 150 %,

(c) une recouvrance ($R_{300}$) à 300 secondes supérieure à 80 %.

Cette composition permet notamment d'obtenir des colorations intenses, résistantes aux agents extérieurs.

EP 1 621 185 A1

## Description

L'invention a pour objet une composition de coloration comprenant, dans un milieu approprié à la coloration des matières kératiniques, un précurseur de colorant et un polymère filmogène aux caractéristiques élastomères particulières, ainsi que le procédé de coloration des matières kératiniques, en particulier les fibres kératiniques, à partir de cette composition.
Depuis longtemps, de nombreuses personnes cherchent à modifier la couleur de leur peau, de leurs cils ou de leurs cheveux et en particulier à masquer leurs cheveux blancs. Pour ce faire, plusieurs technologies ont été développées.
Il est connu de teindre les fibres kératiniques humaines, et en particulier les cheveux, avec des compositions tinctoriales contenant des précurseurs de colorants d'oxydation tels que des bases d'oxydation ou des coupleurs. Les bases d'oxydation sont des composés incolores ou faiblement colorés qui, associés à des produits oxydants, donnent naissance par un processus de condensation oxydative à des composés colorés. Ces colorants sont insolubles et sont piégés à l'intérieur de la fibre capillaire.
On sait également que l'on peut faire varier les nuances obtenues avec ces bases d'oxydation en les associant à des coupleurs ou modificateurs de coloration. La variété des molécules mises en jeu au niveau des bases d'oxydation et des coupleurs permet l'obtention d'une riche palette de couleurs.
Les colorations obtenues présentent une bonne ténacité aux shampooings. Cependant, la réaction d'oxydation se fait à l'aide de produits oxydants tels l'eau oxygénée en milieu basique. Ces agents oxydants attaquent la kératine des cheveux dont les propriétés cosmétiques et mécaniques peuvent se dégrader fortement en cas de colorations répétées ce qui peut entraîner un coiffage ou une mise en forme difficile.
Il est déjà connu d'introduire dans les compositions de coloration d'oxydation des polymères classiquement utilisés pour la formulation des produits capillaires de coiffage. Par exemple, il est connu d'ajouter des polymères filmogènes anioniques, amphotères ou non ioniques, qui conduisent à la formation de films possédant un caractère plus ou moins dur et cassant.
Il existe un besoin dans le domaine de la coloration de composition permettant d'obtenir des colorations intenses, dans des nuances variées, qui présentent une bonne résistance dans le temps et résistent aux agents extérieurs tels que la lumière, le shampooing, la sueur tout en préservant la qualité de la matière kératinique et de sa facilité de coiffage, notamment un bon démêlage, de la douceur et un aspect agréable, non collant, ainsi qu'une facilité d'emploi. Il existe aussi un besoin de disposer de compositions de coloration aptes à former sur ces matières kératiniques un film souple qui suit les mouvements de la peau ou de la chevelure, sans effet de tiraillement, de lourdeur, ou de sensation rigide.
Ce but est atteint par la présente invention qui concerne une composition de coloration comprenant, dans un milieu approprié à la coloration, au moins un précurseur de colorant et au moins un polymère filmogène élastomère choisi de telle sorte que le film obtenu par séchage de ce polymère, à température ambiante et à un taux d'humidité relative de 55 %, présente un profil mécanique défini par au moins :

(a) un taux d'allongement à la rupture ($\varepsilon_r$) supérieur ou égal à 800 %,
(b) une recouvrance instantanée ($R_i$) au moins égale à 75 %, après un allongement de 150 %,
(c) une recouvrance ($R_{300}$) à 300 secondes supérieure à 80 %

Cette composition permet notamment d'obtenir des colorations intenses, résistantes aux agents extérieurs tout en préservant l'intégrité des matières kératiniques et un bon coiffage par la formation d'un film souple, non cassant sur les matières kératiniques humaines, et qui suit leurs mouvements.
Par "au moins un" polymère filmogène élastomère, on entend au sens de l'invention, un ou plusieurs (2, 3 ou plus) polymères filmogènes élastomères.
Par précurseurs de colorant, on entend toute molécule non colorée qui sous l'action d'un agent oxydant donne une espèce colorée qui colore les matières kératiniques.
Un autre objet de la présente invention se rapporte à un procédé de coloration des matières kératiniques, en particulier les fibres kératiniques à partir de la composition de l'invention.
Encore un objet de la présente invention se rapporte à l'utilisation de cette composition pour la coloration des matières kératiniques, en particulier les fibres kératiniques telles que les cheveux.
Au sens de la présente invention, on entend par film obtenu par séchage à température ambiante (22°C ± 2°C) et à un taux d'humidité relative de 55 % ± 5%, le film obtenu dans ces conditions à partir d'un mélange à 6 % de matière active (m.a.) de polymère filmogène élastomère dans un mélange 30 % en poids d'éthanol et de 70 % en poids d'eau, par rapport au poids total alcool+eau, la quantité de mélange étant adaptée pour obtenir dans une matrice en téflon, un film d'épaisseur de 500 μm ± 50 μm. Le séchage est poursuivi jusqu'à ce que le poids du film n'évolue plus, ce qui représente environ 12 jours. Les polymères filmogène solubles ou partiellement solubles dans l'éthanol sont testés dans l'éthanol seul. Les autres polymères sont testés dans l'eau seule, sous forme soluble ou dispersée.

Au sens de la présente invention, le taux d'élongation à la rupture et le taux de recouvrance sont évalués aux moyens des essais décrits ci-après.

Pour effectuer les essais de traction, le film est découpé en éprouvettes de forme rectangulaire, de longueur 80 mm et de largeur 15 mm.

Les essais sont réalisés sur un appareil commercialisé sous l'appellation Lloyd ou commercialisé sous l'appellation Zwick dans les mêmes conditions de températures et d'humidité que pour le séchage, c'est-à-dire une température de 22°C ± 2 °C et un taux d'humidité relative de 50 % ± 5 %.

Les éprouvettes sont étirées à la vitesse de 20mm/min et la distance entre les mors est de 50 ± 1 mm.

Pour déterminer la recouvrance instantanée ($R_i$), on procède comme suit :

- on étire l'éprouvette de 150 % ($\varepsilon_{max}$) c'est-à-dire 1,5 fois sa longueur initiale ($l_0$),
- on relâche la contrainte en imposant une vitesse de retour égale à la vitesse de traction, soit 20mm/min, et on mesure l'allongement de l'éprouvette en pourcentage, après retour à charge nulle ($\varepsilon_i$).

La recouvrance instantanée en % ($R_i$) est donnée par la formule ci-après :

$$R_i = ((\varepsilon_{max} - \varepsilon_i)/ \varepsilon_{max}) \times 100$$

Pour déterminer la recouvrance à 300 secondes, on maintient à contrainte nulle pendant 300 secondes supplémentaires, l'éprouvette ayant subi les opérations précédentes, et on mesure son taux d'allongement en pourcentage ($\varepsilon_{300s}$).

La recouvrance à 300 secondes en % ($R_{300s}$) est donnée par la formule ci-après :

$$R_{300s} = ((\varepsilon_{max} - \varepsilon_{300s})/ \varepsilon_{max}) \times 100$$

De façon avantageuse, le ou les polymères de la composition selon l'invention, éventuellement associé(s) à un agent auxiliaire de filmification, sont tels qu'ils forment, dans les conditions des tests ci-dessus, une film d'allongement à la rupture allant de 800 % à 3000 % ; de recouvrance instantanée de 75 % à 100 % ; et une recouvrance à 300secondes allant de 85 % à 100 %.

Dans les compositions conformes à l'invention, le polymère filmogène élastomère ou le mélange de polymères filmogènes élastomères est, de préférence, présent à une concentration de 0,05 % à 20 % en poids, plus préférentiellement de 0,1 % à 15 % en poids, et par exemple de 0,25 % à 10 % en poids par rapport au poids total de la composition.

De manière avantageuse, le polymère filmogène élastomère est choisi dans le groupe comprenant les polyuréthannes, les alcools polyvinyliques, les polymères comprenant au moins un motif (méth)acrylique, leurs associations. Il peut se présenter sous forme d'homopolymère ou de copolymère. En particulier, il se présente sous forme non réticulée dans la composition.

De préférence, le (ou les) polymères filmogènes élastomères utiles dans la présente invention sont solubles ou hydrodispersibles en milieu aqueux ou hydroalcoolique. En particulier, le ou les polymères filmogènes élastomères sont solubles à au moins 10 g en matière active dans 90 g de milieu aqueux ou hydroalcoolique (contenant 70 % eau et 30 % éthanol), à température ambiante et pression atmosphérique.

Selon un mode de réalisation particulier, le film formé à partir de la composition de l'invention présente une faible sensibilité à l'eau, par exemple en atmosphère d'humidité relative de 30 à 80%, c'est-à-dire que le film garde ses propriétés élastomères pendant plusieurs heures. Il est souple, non cassant et suit bien les mouvements de la peau et/ou de la chevelure. En particulier, entre 30 % et 80 % d'humidité relative, le taux d'allongement à la rupture du film obtenu ne varie pas de plus de 50 % (± 400 %) et/ou sa recouvrance instantanée ne varie pas de plus de 25 % (18,75 %). Autrement dit, entre 30 et 80 % d'humidité relative, le taux d'allongement à la rupture du film obtenu est compris entre 400 % et 1200 % et/ou sa recouvrance instantanée est comprise entre 57 et 93 %.

Les polymères filmogènes élastomères utiles dans la composition de l'invention sont connus de la technique. Ils peuvent par exemple être synthétisés selon la méthode décrite dans la demande de brevet FR 2 815 350.

Les précurseurs de colorants utiles dans la composition de la présente invention sont des bases d'oxydation ou des coupleurs conventionnellement utilisés dans le domaine de la coloration d'oxydation.

A titre de base d'oxydation, on peut notamment citer les paraphénylènediamines, les bis-phénylalkylènediamines, les paraaminophénols, les ortho-aminophénols et les bases hétérocycliques.

Parmi les para-phénylènediamines, on peut citer à titre d'exemple, la para-phénylènediamine, la para-toluènediamine, la 2-chloro para-phénylènediamine, la 2,3-diméthyl para-phénylènediamine, la 2,6-diméthyl para-phénylènediamine, la 2,6-diéthyl para-phénylènediamine, la 2,5-diméthyl para-phénylènediamine, la N,N-diméthyl para-phénylènediamine, la N,N-diéthyl para-phénylènediamine, la N,N-dipropyl para-phénylènediamine, la 4-amino N,N-diéthyl 3-méthyl aniline, la N,N-bis-(β-hydroxyéthyl) para-phénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl para-phénylènediamine, la 2-fluoro para-phénylènediamine, la 2-isopropyl para-phénylènediamine, la N-(β-hydroxypropyl) para-phénylènediamine, la 2-hydroxyméthyl para-phénylènediamine, la N,N-diméthyl 3-méthyl para-phénylènediamine, la N,N-(éthyl, β-hydroxyéthyl) para-phénylènediamine, la N-(β,γ-dihydroxypropyl) para-phénylènediamine, la N-(4'-aminophényl) para-phénylènediamine, la N-phényl para-phénylènediamine, la 2-β-hydroxyéthyloxy para-phénylènediamine, la 2-β-acétylaminoéthyloxy para-phénylènediamine, la N-(β-méthoxyéthyl) para-phénylènediamine, la 4-aminophénylpyrrolidine, la 2-thiényl para-phénylènediamine, le 2-β hydroxyéthylamino 5-amino toluène, la 3-hydroxy 1-(4'-aminophényl)pyrrolidine et leurs sels d'addition avec un acide.

Parmi les para-phénylènediamines citées ci-dessus, la para-phénylènediamine, la para-toluènediamine, la 2-isopropyl para-phénylènediamine, la 2-β-hydroxyéthyl para-phénylènediamine, la 2-β-hydroxyéthyloxy para-phénylènediamine, la 2,6-diméthyl para-phénylènediamine, la 2,6-diéthyl para-phénylènediamine, la 2,3-diméthyl para-phénylènediamine, la N,N-bis-(β-hydroxyéthyl) para-phénylènediamine, la 2-chloro para-phénylènediamine, la 2-β-acétylaminoéthyloxy para-phénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques tels que les 4,5diamino pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs d'addition avec un acide ou avec une base.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2750048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

La ou les bases d'oxydation présentes dans la composition de l'invention sont en général présentent chacune en quantité comprise entre 0,001 à 10 % en poids environ du poids total de la composition tinctoriale, de préférence entre 0,005 et 6 %.

A titre de coupleurs, on peut notamment citer les méta-phénylènediamines, les méta-aminophénols, les méta-diphénols, les coupleurs naphtaléniques, les coupleurs hétérocycliques et leur sels d'addition.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition avec un acide.

Dans la composition de la présente invention, le ou les coupleurs sont chacun généralement présents en quantité comprise entre 0,001 et 10 % en poids environ du poids total de la composition tinctoriale, de préférence entre 0,005 et 6 %.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

La composition tinctoriale conforme à l'invention peut en outre contenir un ou plusieurs colorants directs pouvant notamment être choisis parmi les colorants nitrés de la série benzénique, les colorants directs azoïques, les colorants directs méthiniques. Ces colorants directs peuvent être de nature non ionique, anionique ou cationique.

La composition peut, en outre comprendre un agent auxiliaire de filmification tel qu'un agent plastifiant et/ou un agent facilitant la filmification du ou des polymères élastomères sur les matières kératiniques, et dont la fonction est de modifier les propriétés du ou des polymères élastomères. Un tel agent auxiliaire de filmification peut être choisi parmi tous les composés connus de l'homme du métier comme étant susceptibles de remplir la fonction recherchée, et être notamment choisi parmi les agents plastifiants et les agents de coalescence. Le ou les polymères filmogènes élastomères, éventuellement associés à un agent plastifiant et/ou un agent facilitant la filmification sont aptes à former un film, après évaporation du milieu cosmétique. Cette évaporation peut être faite à l'air libre ou en apportant de la chaleur, par exemple à l'aide d'un séchoir.

Comme exemple d'agent plastifiant et/ou facilitant la filmification sur les matières kératiniques, on peut utiliser ceux décrits dans le document FR-A-2 782 917. En particulier, cet agent est choisi parmi les plastifiants ou agents de coalescence usuels, tels que:

- les glycols et leurs dérivés tels que le diéthylène glycol éthyléther, le diéthylène glycol méthyléther, le diéthylène glycol butyléther ou encore le diéthylène glycol hexyléther, l'éthylène glycol éthyléther, l'éthylène glycol butyléther, l'éthylène glycol hexyléther, le pentylène glycol,
- les esters de glycérol,
- les dérivés de propylène glycol et en particulier le propylène glycol phényléther, le propylène glycol diacétate, le dipropylène glycol butyléther, le tripropylène glycol butyléther, le propylène glycol méthyléther, le dipropylène glycol éthyléther, le tripropylène glycol méthyléther et le diéthylène glycol méthyléther, le propylène glycol butyléther,
- les esters d'acides notamment carboxyliques, tels que des citrates, des phtalates, des adipates, des carbonates, des tartrates, des phosphates, des sébaçates,

- leurs mélanges.

La quantité d'agent plastifiant et/ou d'agent de filmification peut être choisie par l'homme du métier sur base de ses connaissances générales, de manière à obtenir un système polymérique (polymères élastomères + agent plastifiant et/ou agent de filmification) conduisant à un film ayant les propriétés mécaniques souhaitées, tout en conservant à la composition les propriétés cosmétiques recherchées. En pratique, cette quantité varie de 0,01 % à 25 % du poids total de la composition et mieux de 0,01 % à 15 %.

La composition tinctoriale conforme l'invention peut également renfermer divers adjuvants utilisés classiquement dans les compositions pour la teinture des cheveux, tels que des agents tensio-actifs anioniques, cationiques, non-ioniques, amphotères, zwitterioniques ou leurs mélanges, des polymères anioniques, cationiques, non-ioniques, amphotères, zwitterioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones, des agents filmogènes autres que ceux de l'invention et en particulière des polymères fixants non ioniques, cationiques, anioniques, amphotères, des agents conservateurs, des agents opacifiants.

Le milieu approprié pour la coloration appelé aussi support de teinture est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en $C_1$-$C_4$, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante :

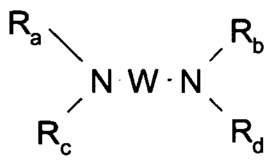

(II)

dans laquelle W est un reste propylène éventuellement substitué par un groupement hydroxyle ou un radical alkyle en $C_1$-$C_4$ ; $R_a$, $R_b$, $R_c$ et $R_d$, identiques ou différents, représentent un atome d'hydrogène, un radical alkyle en $C_1$-$C_4$ ou hydroxyalkyle en $C_1$-$C_4$.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Le procédé de la présente invention est un procédé dans lequel on applique sur les fibres la composition selon la présente invention telle que définie précédemment, en présence d'un agent oxydant pendant un temps suffisant pour développer la coloration désirée. La couleur peut être révélée à pH acide, neutre ou alcalin et l'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

Les agents oxydants classiquement utilisés sont par exemple le peroxyde d'hydrogène, le peroxyde d'urée,

les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

La composition tinctoriale selon l'invention peut se présenter sous des formes diverses, telles que sous forme de liquides, de crèmes, de gels, ou sous toute autre forme appropriée pour réaliser une teinture des matières kératiniques et notamment des cheveux humains.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Les exemples qui suivent illustrent la présente invention sans toutefois en limitée la portée.

**EXEMPLES**

Les quantités sont données en pourcentage massique et M.A. signifie matière active.

**Exemple 1 :**

On prépare une composition de teinture qui comprend :

| | |
|---|---|
| Polyuréthane (NMDEA1) / PTMO 29002) / IPD13) - 3 / 1/ 4) | 2 % MA |
| paraphénylènediamine | 0,25 g |
| Résorcine | 0,25g |
| Milieu de coloration | (*) |
| Eau déminéralisée | Qsp 100 g |
| 1) - N-méthyldiéthanolamine<br>2) - Poly (tétraméthylène oxyde) ayant une masse moyenne en poids de 2900<br>3) - Isophoronediisocianate | |

Le polymère a été préparé selon la méthode de synthèse décrite dans la demande de brevet FR 2 815 350.

Le copolymère de polyuréthane présente les caractéristiques suivantes, mesurées comme ci-dessus :

- $\varepsilon_r$ = 1500 %
- $R_i$ = 82 %
- $R_{300}$ = 92 %
- Soluble dans l'eau à au moins 10 g par litre

<u>**Milieu de coloration (*)**</u>

- Alcool oléique polyglycérolé à 2 moles de glycérol 4 g
- Alcool oléique polyglycérolé à 4 moles de glycérol( 78 % M.A. ) 5,69 g M.A.
- Acide oléique 3,0 g
- Amine oléique 2 OE commercialisée sous la dénomination d'ETHOMEEN 012 par la société AKZO 7 g

- Laurylamino succinamate de diéthylaminopropyle, sel de sodium à 55% M.A. 3 g M.A.
- Alcool oléique 5 g
- Diéthanolamide d'acide oléique 12 g
- Propylène glycol 3,5 g
- Alcool éthylique 7,0 g
- Dipropylène glycol 0,5 g
- Monométhyléther de propylène glycol 9 g
- Métabisulfite de sodium en solution aqueuse à 35% M.A. 0,455 g M.A.
- Acétate d'ammonium 0,8 g
- Antioxydant, séquestrant q.s.
- Parfum, conservateur q.s.
- Ammoniaque à 20% de NH3 10 ,2 g

Au moment de l'emploi, la composition est mélangée avec un poids égal d'eau oxygénée à 20 volumes (6% en poids). Le mélange obtenu est appliqué sur des mèches de cheveux naturels gris à 90 % de blancs. Après 30 min de pose, les mèches sont rincées, lavées avec un shampooing standard, rincées à nouveau puis séchées.
On obtient ainsi une coloration des fibres kératiniques en blond foncé.

**Exemple 2 :**

On prépare une composition de teinture qui comprend :

| | |
|---|---|
| Polyuréthane (NMDEA1) / PTMO 29002) / IPDI3) - 3 / 1/ 4) | 2 % MA |
| Para-aminophénol | 0,4 g |
| 2 méthyl 5 aminophénol | 0,5g |
| Milieu de coloration | (*) |
| Eau déminéralisée | Qsp 100 g |
| 1) - N-méthyldiéthanolamine<br>2) - Poly (tétraméthylène oxyde) ayant une masse moyenne en poids de 2900<br>3) - Isophoronediisocianate | |

La composition est mélangée à de l'eau oxygénée et appliquée sur les fibres kératiniques selon la méthode décrite dans l'exemple 1.
On obtient ainsi une coloration des fibres en blond foncé cuivré.

**Exemple 3 :**

On prépare une composition de teinture qui comprend :

| | |
|---|---|
| Polyuréthane (NMDEA1) / PTMO 29002) / IPDI3) - 3 / 1/ 4) | 2 % MA |
| 4,5-diamino 1-hydroxyéthyl pyrazole, sulfate | 1,0 g |
| 2 méthyl 5 aminophénol | 0,7g |
| Milieu de coloration | (*) |
| Eau déminéralisée | Qsp 100 g |
| 1) - N-méthyldiéthanolamine<br>2) - Poly (tétraméthylène oxyde) ayant une masse moyenne en poids de 2900 | |

Suite de tableau

| |
|---|
| 3) - Isophoronediisocianate |

La composition est mélangée à de l'eau oxygénée et appliquée sur les fibres kératiniques selon la méthode décrite dans l'exemple 1.
On obtient ainsi une coloration des fibres en châtain clair cuivré.

## Revendications

1. Composition de coloration comprenant, dans un milieu approprié à la coloration, au moins un précurseur de colorant et au moins un polymère filmogène élastomère choisi de telle sorte que le film obtenu par séchage de ce polymère, à température ambiante et à un taux d'humidité relative de 55 %, présente un profil mécanique défini par au moins :

   (a) un taux d'allongement à la rupture ($\varepsilon_r$) supérieur ou égal à 800 %,
   (b) une recouvrance instantanée ($R_i$) au moins égale à 75 %, après un allongement de 150 %,
   (c) une recouvrance ($R_{300}$) à 300 secondes supérieure à 80 %.

2. Composition selon la revendication 1, **caractérisée en ce que** le polymère filmogène élastomère est soluble dans un milieu aqueux ou hydroalcoolique.

3. Composition selon la revendication 1 ou 2, **caractérisée en ce** le polymère est tel qu'entre 30 % et 80 % d'humidité relative, le taux d'allongement à la rupture du film obtenu est comprise entre 400 % et 1200 % et/ou sa recouvrance instantanée est comprise entre 57 et 93 %.

4. Composition selon l'une quelconque des revendications précédentes, **caractérisée en ce que** le polymère filmogènes élastomère ou le mélange de polymères filmogènes élastomères est présent à une concentration allant de 0,05 % à 20 % en poids, plus préférentiellement de 0,1 % à 15 % en poids.

5. Composition selon l'une quelconque des revendications précédentes, **caractérisée en ce que** le polymère filmogène est choisi dans le groupe comprenant les polyuréthannes, les alcools polyvinyliques, les polymères comprenant au moins un motif (méth)acrylique, leurs associations.

6. Composition selon l'une quelconque des revendications précédentes dans laquelle le ou les précurseurs de colorants sont choisis parmi les bases d'oxydation.

7. Composition selon la revendication 6 dans laquelle les bases d'oxydation sont choisies parmi les paraphénylènediamines, les bis-phénylalkylènediamines, les paraaminophénols, les ortho-aminophénols et les bases hétérocycliques.

8. Composition selon la revendication 6 dans laquelle les bases d'oxydation sont choisies parmi la para-phénylènediamine, la para-toluènediamine, la 2-chloro para-phénylènediamine, la 2,3-diméthyl para-phénylènediamine, la 2,6-diméthyl para-phénylènediamine, la 2,6-diéthyl para-phénylènediamine, la 2,5-diméthyl para-phénylènediamine, la N,N-diméthyl para-phénylènediamine, la N,N-diéthyl para-phénylènediamine, la N,N-dipropyl para-phénylènediamine, la 4-amino N,N-diéthyl 3-méthyl aniline, la N,N-bis-(β-hydroxyéthyl) para-phénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl para-phénylènediamine, la 2-fluoro para-phénylènediamine, la 2-isopropyl para-phénylènediamine, la N-(β-hydroxypropyl) para-phénylènediamine, la 2-hydroxyméthyl para-phénylènediamine, la N,N-diméthyl 3-méthyl para-phénylènediamine, la N,N-(éthyl, β-hydroxyéthyl) para-phénylènediamine, la N-(β,γ-dihydroxypropyl) para-phénylènediamine, la N-(4'-aminophényl) para-phénylènediamine, la N-phényl para-phénylènediamine, la 2-β-hydroxyéthyloxy para-phénylènediamine, la 2-β-acétylaminoéthyloxy para-phénylènediamine, la N-(β-méthoxyéthyl) para-phénylènediamine, la 4-aminophénylpyrrolidine, la 2-thiényl para-phénylènediamine, le 2-β hydroxyéthylamino 5-amino toluène, la 3-hydroxy 1-(4'-aminophényl)pyrrolidine et leurs sels d'addition avec un acide.

9. Composition selon la revendication 6 dans laquelle les bis-phénylalkylènediamines sont choisies parmi le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

**10.** Composition selon la revendication 6 dans laquelle les para-aminophénols sont choisis parmi le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

**11.** Composition selon la revendication 6 dans laquelle les ortho-aminophénols sont choisis parmi le 2-amino phénol, le 2-amino 5-méthyl phénol, le 2-amino 6-méthyl phénol, le 5-acétamido 2-amino phénol, et leurs sels d'addition avec un acide-.

**12.** Composition selon la revendication 6 dans laquelle les bases hétérocycliques sont choisies parmi les dérivés pyridiniques, les dérivés pyrimidiniques et les dérivés pyrazoliques.

**13.** Composition selon la revendication 12 dans laquelle les dérivés pyridiniques sont choisis parmi la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ainsi que leurs d'addition avec un acide ou avec une base.

**14.** Composition selon la revendication 12 dans laquelle les dérivés pyrimidiniques sont choisis parmi la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]-pyrimidin-7-yl)-(2-hydroxy-éthyl)-amino]-éthanol, le 2-[(7-amino-pyrazolo[1,5-a]-pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

**15.** Composition selon la revendication 12 dans laquelle les dérivés pyrazoliques sont choisis parmi le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

**16.** Composition selon l'une quelconque des revendications précédentes dans laquelle la ou les bases d'oxydation représentent de 0,001 à 10 % en poids environ du poids total de la composition tinctoriale.

**17.** Composition selon l'une quelconque des revendications précédentes dans laquelle le ou les précurseur de colorant

sont des coupleurs choisis parmi les métaphénylènediamines, les méta-aminophénols, les métadiphénols, les naphtols et les coupleurs indoliques, indoliniques, pyridiniques, indazoliques, pyrazolo[1,5-b]-1,2,4-triazole, pyrazolo[3,2-c]-1,2,4-triazole, benzimidazole, benzothiazole, benzoxazole, 1,3-benzodioxole et les pyrazolones, et leurs sels d'addition avec un acide.

**18.** Composition selon la revendication 17 dan slaquelle le ou les coupleurs sont choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 2-méthyl-1-naphtol, le 6-hydroxy indole, le 4-hydroxy indole, le 4-hydroxy N-méthyl indole, la 6-hydroxy indoline, la 6-hydroxybenzomorpholine, la 3,5 diamino 2,6-diméthoxy pyridine, le 1-N-( β hydroxyéthyl)amino 3,4 méthylènedioxy benzène, le 2,6 bis(β hydroxyéthylamino)toluène, la 2,6-dihydroxy 4-méthyl pyridine, le 1-H 3-méthyl pyrazole 5-one, le 1-phényl 3-méthyl pyrazole 5-one, et leurs sels d'addition avec un acide.

**19.** Composition selon l'une quelconque des revendications 1 à 18 dans laquelle le ou les coupleurs représentent de 0,0001 à 10 % en poids environ du poids total de la composition tinctoriale.

**20.** Composition selon l'une quelconque des revendications précédentes comprenant de plus un agent oxydant.

**21.** Composition selon la revendication 20 dans laquelle l'agent oxydant est choisi parmi le peroxyde d'hydrogène, le peroxyde d'urée, les bromates de métaux alcalins, les persels, les peracides et les enzymes oxydases.

**22.** Procédé de coloration des matières kératiniques qui comprend l'application sur la matière kératinique d'une composition telle que définie aux revendications 1 à 19 en présence d'un agent oxydant pendant un temps suffisant pour colorer les matières kératiniques.

**23.** Procédé selon la revendication 22 pour la coloration des fibres kératiniques.

**24.** Utilisation d'une composition selon l'une quelconque des revendications 1 à 21 pour la teinture des fibres kératiniques.

Office européen des brevets

# RAPPORT DE RECHERCHE EUROPEENNE

Numéro de la demande
EP 05 29 1420

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | FR 2 786 391 A1 (L'OREAL) 2 juin 2000 (2000-06-02) * revendications 1,7 * ----- | 1-24 | A61K7/13 |
| X | FR 2 786 392 A1 (L'OREAL) 2 juin 2000 (2000-06-02) * revendications 1,8 * ----- | 1-24 | |
| X | FR 2 815 350 A1 (L'OREAL) 19 avril 2002 (2002-04-19) * le document en entier * ----- | 1-24 | |

DOMAINES TECHNIQUES RECHERCHES (Int.Cl.7)
A61K

Le présent rapport a été établi pour toutes les revendications

1

| Lieu de la recherche | Date d'achèvement de la recherche | Examinateur |
|---|---|---|
| Munich | 16 septembre 2005 | Yon, J-M |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
..........
& : membre de la même famille, document correspondant

EPO FORM 1503 03.82 (P04C02)

**ANNEXE AU RAPPORT DE RECHERCHE EUROPEENNE RELATIF A LA DEMANDE DE BREVET EUROPEEN NO.** EP 05 29 1420

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

16-09-2005

| Document brevet cité au rapport de recherche | Date de publication | Membre(s) de la famille de brevet(s) | Date de publication |
|---|---|---|---|
| FR 2786391 A1 | 02-06-2000 | AU 745846 B2 | 11-04-2002 |
| | | AU 1276700 A | 13-06-2000 |
| | | BR 9907723 A | 17-10-2000 |
| | | CN 1295459 A | 16-05-2001 |
| | | EP 1051146 A1 | 15-11-2000 |
| | | WO 0030594 A1 | 02-06-2000 |
| | | JP 2002530310 T | 17-09-2002 |
| | | JP 2004307516 A | 04-11-2004 |
| | | PL 341980 A1 | 07-05-2001 |
| | | RU 2183449 C2 | 20-06-2002 |
| | | US 6391292 B1 | 21-05-2002 |
| FR 2786392 A1 | 02-06-2000 | AU 746140 B2 | 18-04-2002 |
| | | AU 1276600 A | 13-06-2000 |
| | | BR 9907724 A | 17-10-2000 |
| | | CN 1295458 A | 16-05-2001 |
| | | EP 1051145 A1 | 15-11-2000 |
| | | WO 0030593 A1 | 02-06-2000 |
| | | JP 2002530309 T | 17-09-2002 |
| | | PL 341932 A1 | 07-05-2001 |
| | | RU 2200534 C2 | 20-03-2003 |
| | | US 6497864 B1 | 24-12-2002 |
| FR 2815350 A1 | 19-04-2002 | AU 9569201 A | 29-04-2002 |
| | | EP 1326908 A1 | 16-07-2003 |
| | | WO 0232978 A1 | 25-04-2002 |
| | | JP 2004511637 T | 15-04-2004 |
| | | US 2004052753 A1 | 18-03-2004 |

EPO FORM P0460

Pour tout renseignement concernant cette annexe : voir Journal Officiel de l'Office européen des brevets, No.12/82